# EUROPÄISCHE PATENTSCHRIFT

(11) **EP 1 363 618 B1**
(45) Veröffentlichungstag und Bekanntmachung des Hinweises auf die Patenterteilung: **03.05.2006**
(21) Anmeldenummer: 02719715.1
(22) Anmeldetag: 23.01.2002
(51) Int. Cl.: A61K 31/34, A61K 31/38, A61K 31/40, A61P 25/00

(54) **PHARMAZEUTISCHE ZUSAMMENSETZUNG ZUR BEHANDLUNG VON MULTIPLE SKLEROSE**
PHARMACEUTICAL COMPOSITION FOR TREATING MULTIPLE SCLEROSIS
COMPOSITION PHARMACEUTIQUE POUR TRAITER LA SCLEROSE EN PLAQUES

(30) Priorität: 26.01.2001 DE 10103506
(43) Veröffentlichungstag der Anmeldung: 26.11.2003
(73) Patentinhaber: Neu, Ingo, S., 71116 Grätringen 2 (Rohrau) (DE)
(72) Erfinder: Neu, Ingo, S., 71116 Grätringen 2 (Rohrau) (DE)
(74) Vertreter: HOFFMANN EITLE
(86) Internationale Anmeldenummer: PCT/EP2002/000653
(87) Internationale Veröffentlichungsnummer: WO 2002/062332

(56) Entgegenhaltungen:
- EP-A- 0 279 263
- US-A- 5 093 356
- PROSIEGEL M ET AL: "SUPPRESSION OF EXPERIMENTAL AUTOIMMUNE ENCEPHALOMYELITIS BY SULFASALAZINE" ACTA NEUROLOGICA SCANDINAVICA, Bd. 81, Nr. 3, 1990, Seiten 237-238, XP001080417
- PROSIEGEL M ET AL: "SUPPRESSION OF EXPERIMENTAL AUTOIMMUNE ENCEPHALOMYELITIS BY DUAL CYCLO-OXYGENASE AND 5-LIPOXYGENASE INHIBITION" ACTA NEUROLOGICA SCANDINAVICA, MUNKSGAARD, COPENHAGEN, DK, Bd. 79, Nr. 3, 1. März 1989 (1989-03-01), Seiten 223-226, XP002043517 ISSN: 0001-6314
- COHN, J.: "ZILEUTON (A-64077): A 5-Lipoxygenase Inhibitor" NONSTEROIDAL ANTI-INFLAMMATORY DRUGS (2 ED.), 1994, Seiten 367-390, XP001079053

## Beschreibung

Diese Erfindung betrifft die Verwendung von organischen Verbindungen zur Herstellung einer pharmazeutischen Zusammensetzung zur Behandlung von Multiple Sklerose beim Menschen.

### Hintergrund der Erfindung

Multiple Sklerose (Encephalomyelitis disseminata) (MS) ist eine der häufigsten Nervenkrankheiten. Ihre Ursachen sind noch nicht bekannt. Bis heute gibt es noch kein kausales Heilungsverfahren, aber es wurden viele Versuche unternommen, um die Auswirkungen der Krankheit zu mindern. Dabei hat sich die zeitlich begrenzte Behandlung der schubweisen Verschlechterung der MS mit Cortisonpräparaten bewährt.

Trotz intensiver Behandlungsversuche zur Rezidivprophylaxe der MS mit immunsuppressiv wirksamen Medikamenten wie Imurek® und zur Immunmodulation mit β-Interferonpräparaten wie Betaferon® ist noch kein überzeugender Durchbruch gelungen, um das Fortschreiten der MS aufzuhalten.

In Nordeuropa, Kanada und den nördlichen Staaten der USA ist MS die häufigste fortschreitende Erkrankung des zentralen Nervensystems. Das Erkrankungsalter liegt zwischen dem 20. und 40. Lebensjahr, Frauen sind etwas häufiger betroffen als Männer.

Die chronische Entzündungsreaktion bei der MS ist durch komplexe Interaktionen zwischen zellulären und humoralen Elementen des Immunsystems, Adhäsionsmolekülen auf Endothelzellen und Entzündungszellen sowie eine Reihe von niedermolekularen Entzündungsmediatoren geprägt. Von den Entzündungsmediatoren spielen die Zytokine eine wichtige Rolle.

Die Symptome der MS sind vielfältig und umfassen im Prodromalstadium unter anderem pseudoneurasthenische Symptome, Hirnnervenausfälle (retrobulbäre Neuritis, Augenmuskellähmung, skandierende Sprache), spastische Lähmungen, Kleinhirnsymptome, Sensibilitäts-, Blasen- und Mastdarmstörungen; Euphorie, eine später dementielle Entwicklung ist möglich.

Die Ätiologie des chronisch-entzündlichen Geschehens ist trotz intensiver Forschung unklar geblieben. In den letzten zwei Jahrzehnten konnten durch pathologisch-anatomische und immunologische Studien an Erkrankten sowie durch experimentelle Arbeiten am Tiermodell der MS, der experimentell allergischen Encephalomyelitis (EAE), wesentliche pathogenetische Mechanismen aufgeklärt werden. Es wird davon ausgegangen, daß T-Zellen vermittelte Autoimmunreaktionen gegen Myelin-Antigene eine bedeutende Rolle spielen.

Da die Ursache der MS bis heute unbekannt geblieben ist, hat sich die Behandlung dieser Krankheit noch stets als problematisch gezeigt. Die kontroverse Situation der MS-Therapie (Bauer H.I.: "Nervenarzt", 54 (1983), 400 - 405) zeigt sich bei mehr als 100 Versuchen der Behandlung, welche zumindest zeitweise in der Medizin ausgeübt wurden, einschließlich unkonventioneller Verfahren und Verfahren von Außenseitern.

Manche Autoren haben eine infektiöse Ursache der MS postuliert und eine Hypothese nimmt an, daß Viren eine signifikante Rolle spielen könnten. Bei der Annahme einer viralen Infektion zeigten verabreichte Therapeutika, insbesondere Amantadin oder Methisazon, keine überzeugenden Ergebnisse. Die Verwendung von Interferon wurde auch in vielen Fällen diskutiert, aber es zeigten sich keine überzeugenden Resultate der Behandlung hinsichtlich der Krankheitsprogredienz.

Andere Prinzipien der Behandlung basieren auf metabolischen Theorien. Annahmen möglicher Protein-Metabolismusstörungen basieren auf der Anwesenheit einer Zahl von biogenischen Aminen, welche bei gesunden Personen nicht gefunden werden konnten.

Die Metabolismushypothese basiert auf Versuchen mit hoch ungesättigten Fettsäuren (Omega-Fettsäuren), da bei höherer Pflanzenöl- oder Fischaufnahme eine niedrigere MS-Prävalenz beobachtet wird.

Andere Untersuchungen basieren auf der Thrombozyten-Aggregation, welche im Fall von MS-Patienten während des Schubes signifikant erhöht ist. Dies hat zu Behandlungsversuchen mit Aggregations-inhibierenden therapeutischen Substanzen geführt.

Schließlich wurde versucht Corticosteroide und Immunsuppressoren in therapeutischen Verfahren einzusetzen, welche in der akuten frühen Phase die Intensität und Dauer der entsprechenden Schubphasen abschwächen können. Aufgrund der großen Nebeneffekte der Corticosteroide, ACTH, usw. ist nur eine begrenzte zeitliche Anwendung möglich, obwohl dies weitere Attacken nicht verhindern kann. Aktuell hat sich die Pulsetherapie mit Cortisonpräparaten im Schub durchgesetzt.

Außer den oben erwähnten Therapien sind eine Reihe von unkonventionellen Verfahren versucht worden, einschließlich Akupunktur, besondere Diäten, Megavitamintherapien, usw. Außerdem wurde vor 15 bis 20 Jahren die Sauerstoff-Überdrucktherapie propagiert (B. Fischer, New England J. Med. 303 (1983) 181 - 186).

Die Schwierigkeiten der MS-Forschung und -Therapie basieren einerseits darauf, daß die Ursachen und Auslöser der Krankheit nicht bekannt sind. Es gibt zwar ein Tiermodell zum Testen von Medikamenten, die experimentelle allergische Encephalomyelitis Encephalomyelitis (EAE), die Resultate können aber nicht zwangsläufig auf Menschen übertragen werden.

In Acta Neurol. Scand. 75 (1987), 361-363 (Neu, et al.) wurde die Freisetzung von Leukotrienen aus Neutrophilen und aus Suspensionen von Thrombozyten und Neutrophilen in Reaktion auf Ionophor A23187 gemessen. Basierend auf den Resultaten dieser wissenschaftlichen Untersuchung wurde postuliert, daß die Freisetzung des Sulfidopeptids Leukotriene bei der perivenulären Plaque-Bildung bei der MS involviert sein könnte. Dort wird weiter ausgeführt, daß wenn diese Ergebnisse für eine größere Zahl von MS-Kranken und Vergleichen mit anderen neurologischen Krankheiten und in EAE-Versuchen bestätigt werden könnten, würde die Suppression der Leukotriensynthese durch Medikamente ein erfolgversprechendes therapeutisches Konzept darstellen. Aus dieser Literaturstelle kann geschlossen werden, daß die wissenschaftlichen Beobachtungen nicht in einer repräsentativen Zahl an Patienten durchgeführt wurden, um daraus Schlüsse ziehen zu können. Obwohl Vergleiche mit anderen neurologischen Krankheiten vorgeschlagen wurden, wurde die genaue Genese dieser Krankheiten nicht genannt. Außerdem wurden Tests an EAE-Tieren als notwendig erachtet, obwohl bekannt ist, daß die Resultate dieser Testsysteme nur begrenzt auf MS-Erkrankte übertragbar sind. Dies wurde letztlich wieder bestätigt, z.B. beim Antimetaboliten Azothioprin (Imurek®), der vielversprechende Indikationen im EAE-Tiertest zeigte, aber bei der Behandlung von MS-Patienten nicht überzeugte.

Weiterhin wurde versucht, über eine pharmakologische Blockade mit Leukotrienbiosyntheseinhibitoren und Leukotrienrezeptorantagonisten die Progression der MS zu beeinflussen (Acta Neurol. Scand. 81 (1990), 237-238). Im Tiermodell konnten die entzündlichen Reaktionen von MS über eine pharmakologische Blockade von Leukotrien C₄ mit chemischen Leukotrienhemmern und Sulphosalazine unterdrückt werden. Diese im Tiermodell erfolgreichen Sulphosalazine (Azulfidine®) wurden in einer klinischen Studie an MS-Patienten getestet. In den ersten 18 Monaten der Behandlung mit Azulfidine konnte in einer USkanadischen Doppelblindstudie ein ungewöhnlich günstiger Effekt objektiviert werden (Neurology 51 (1998), 1342-1352). Im weiteren Verlauf ließ sich dieser Effekt jedoch nicht mehr nachweisen. Hier zeigt sich auch wieder das Problem, der begrenzten Übertragbarkeit der Resultate aus Testsystemen an Tieren auf MS-Erkrankte.

Eine weitere Studie versuchte ebenfalls auf eine leukotrienhemmende Substanz zurückzugreifen (Arzneim.-Forsch./Drug Res. 48(I), 6, 668-674 (1998)). Bei dieser Studie wurde die Boswellia-Säure (Boswellia serrata) getestet, ein Bestandteil des Weihrauchharzes, der vor allem bei der rheumatoiden Polyarthritis, bei Asthma bronchiale und der Colitis ulcerosa eingesetzt wird. In vitro zeigte sich ein guter leukotrienhemmender Effekt..Jedoch bei der tierexperimentellen Untersuchung der Boswellia-Säure konnten keine Erfolge erzielt werden. Damit erübrigte sich ein Behandlungsversuch bei MS-Kranken.

Zusammenfassend zeigt sich, daß aus der Literatur kein schlüssiges Verfahren erkennbar ist, welche spezifischen Wirkstoffe in einer erfolgreichen Therapie von MS verwendet werden können.

### Aufgabe der Erfindung

Es ist eine Aufgabe der Erfindung, einen aktiven Wirkstoff für die Herstellung einer pharmazeutischen Zusammensetzung für die Behandlung von MS beim Menschen zur Verfügung zu stellen.

### Erfindung

Die Aufgabe dieser Erfindung wird gelöst durch die Verwendung einer Verbindung der allgemeinen Formel I und/oder einem pharmazeutisch verträglichen Salz davon als aktiven Wirkstoff für die Herstellung einer pharmazeutischen Zusammensetzung für die Behandlung von MS beim Menschen: 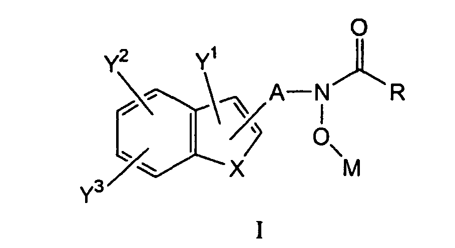 worin:
X: NCH₃, O oder S bedeutet,
A: unsubstituiertes C₁₋₄-Alkylen oder substituiertes C₁₋₄-Alkylen, das einfach oder mehrfach mit Substituenten unabhängig ausgewählt aus CH₃, CH₂CH₃, CH₂CH₂CH₃ und CH(CH₃)₂ substituiert ist, bedeutet,
M: H; Benzoyl; unsubstituiertes C₁₋₁₂-Alkanoyl; substituiertes C₁₋₁₂-Alkanoyl, das einfach oder mehrfach mit Substituenten unabhängig ausgewählt aus CH₃, CH₂CH₃, F und Cl, substituiert ist; unsubstituiertes C₁₋₁₂-Alkyl; oder substituiertes C₁₋₁₂₋Alkyl, das einfach oder mehrfach mit Substituenten unabhängig ausgewählt aus CH₃, CH₂CH₃, F und Cl, substituiert ist; bedeutet,
R: CH₃, CH₂CH₃ oder NR¹R² bedeutet, worin R¹ und R² unabhängig voneinander H, CH₃ oder CH₂CH₃ bedeuten, und
Y¹, Y² und Y³: unabhängig voneinander ausgewählt sind aus H, CH₃, CH₂CH₃, F und Cl.

Überraschend wurde gefunden, daß bei Gabe einer Verbindung der allgemeinen Formel I und/oder eines pharmazeutisch verträglichen Salzes und/oder Hydrats davon an Patienten, die an Multiple Sklerose erkrankt waren, Schubfrequenz und chronische Progredienz zurückgingen. Dieses Ergebnis ist unerwartet, da die Ursachen, die Symptome und das Verhalten von MS und der daran erkrankten Personen entweder unbekannt oder sehr komplex sind.

Bevorzugte erfindungsgemäß verwendete Verbindungen der Formel I schließen solche ein, worin, unabhängig oder in einer oder mehrerer Kombinationen:
M: H bedeutet,
mindestens zwei der Y¹, Y² und Y³: H bedeuten,
A: CH₂ oder CHCH₃ bedeutet, und
X: S bedeutet.

Der erfindungsgemäß verwendete aktive Wirkstoff kann als solcher und/oder in der Form pharmazeutisch verträglicher Salze davon verwendet werden.

Wenn sterische Isomere, wie optische Isomere oder Konformationsisomere, oder Hydrate und Komplexe, von den erfindungsgemäßen Verbindungen existieren, schließt die vorliegende Erfindung jedes und alle dieser ein. Die Verbindungen der vorliegenden Erfindung und die Verfahren zu ihrer Herstellung sind z.B. in der US-PS 4 873 259 offenbart.

Beispiele der erfindungsgemäß verwendeten Verbindungen der allgemeinen Formel I sind:
N-Hydroxy-N-(1-benzo[b]thien-2-ylethyl)-acetamid,
1- (1-Benzo [b] thien-2-ylethyl) -1-hydroxyharnstoff,
N-Hydroxy-N-(1-benzo[b]thien-2-ylethyl)-N'-methyl-harnstoff,
N-Hydroxy-N-(1-benzo[b]thien-2-ylethyl)-N',N'-dimethyl-harnstoff,
N-Hydroxy-N-benzo [b] thien-2-ylmethyl-harnstoff,
N-Hydroxy-N-benzo [b] thien-2-ylmethyl-N'-methyl-harnstoff,
N-Hydroxy-N-benzo[b]thien-2-ylmethyl-N',N'-dimethyl-harnstoff,
N-Hydroxy-N-(1-benzo[b]thien-3-ylethyl)-acetamid,
N-Hydroxy-N-(1-benzo[b]thien-3-ylethyl)-harnstoff,
N-Hydroxy-N-[1-(3-methylbenzo[b]thien-2-yl)-ethyl]-harnstoff,
N-Hydroxy-N-(1-benzo[b]fur-2-ylethyl)-acetamid,
N-Hydroxy-N-(1-benzo[b]fur-2-ylethyl)-harnstoff,
N-Hydroxy-N-[1-(1-methylindol-3-yl)-ethyl]-acetamid,
N-Hydroxy-N-[1-(1-methylindol-3-yl) -ethyl]-harnstoff,
N-Hydroxy-N- [1-(1-methylindol-3-yl)-ethyl]-N'-methylharnstoff,
N-Hydroxy-N-[1-(5-chlorbenzo[b]fur-2-yl)-ethyl]-harnstoff,
N-Hydroxy-N- [1- (5-fluorbenzo [b] thien-2-yl) -ethyl] -harnstoff.

Bevorzugt ist die erfindungsgemäße Verwendung von:
N-Hydroxy-N-(1-benzo[b]thien-2-ylethyl)-acetamid,
1-(1-Benzo[b]thien-2-ylethyl)-1-hydroxyharnstoff,
N-Hydroxy-N-(1-benzo[b]thien-2-ylethyl)-N'-methyl-harnstoff, oder
N-Hydroxy-N-(1-benzo[b]thien-2-ylethyl)-N',N'-dimethylharnstoff.

Besonders bevorzugt ist die erfindungsgemäße Verwendung von 1-(1-Benzo[b]thien-2-ylethyl)-1-hydroxyharnstoff, im folgenden auch Zileuton genannt: 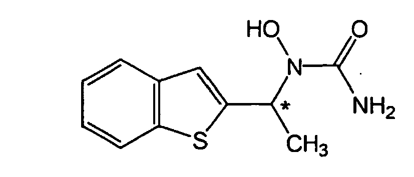 Zileuton kann entweder als racemisches Gemisch der R(+)- und S(-)-Enantiomere oder in Form seiner reinen Enantiomere eingesetzt werden. Bevorzugt ist die Verwendung des Racemats.

Üblicherweise wird der aktive Wirkstoff in einer Menge von 0,001 bis 2,0 mg, bevorzugt in einer Menge von 0,01 bis 2,0 mg, noch bevorzugter in einer Menge von 0,1 bis 1,0 mg, besonders bevorzugt in einer Menge von 600 mg pro Dosis verabreicht. Die Verabreichung geschieht ein- bis fünfmal täglich, bevorzugt viermal täglich.

### Therapeutische Darreichungsformen

Die Herstellung von pharmazeutischen Zusammensetzungen mit einem Gehalt einer Verbindung der allgemeinen Formel I und/oder einem pharmazeutisch verträglichen Salz davon bzw. deren Einsatz bei der erfindungsgemäßen Verwendung erfolgt in üblicher Weise mittels geläufigen pharmazeutischtechnologischen Verfahren. Vorteilhaft ist z. B. die Verarbeitung zusammen mit geeigneten, pharmazeutisch verträglichen Hilfs- und/oder Trägerstoffen zu den für die verschiedenen Indikationen und Applikationsorten geeigneten Arzneiformen. Von Vorteil kann ebenfalls sein, wenn das Arzneimittel weitere Wirkstoffe umfaßt.

Eine wichtige systemische Applikationsform ist die perorale Verabreichung als Tabletten, Hart- oder Weichgelatinekapseln, Dragees, Pulver, Pellets, Mikrokapseln, Oblongkomprimate, Granulate, Kautabletten, Lutschpastillen, Kaugummi, Sachets oder Globuli.

Als Hilfsstoffe für die Herstellung von pharmazeutischen Zusammensetzungen zur peroralen Verabreichung werden beispielsweise Gegenklebe- und Schmier- und Trennmittel, Dispergiermittel, wie z.B. flammendisperses Siliciumdioxid, Sprengmittel, wie z.B. verschiedene Stärkearten, PVP, Celluloseester als Granuliermittel, wie z.B. wachsartige und oder polymere Stoffe auf Eudragit®, Cellulose oder Cremophor®-Basis eingesetzt.

Antioxidantien, Süßungsmittel, wie z.B. Saccharose, Xylit oder Mannit, Geschmackskorrigentien, Aromastoffe, Konservierungsmittel, Farbstoffe, Puffersubstanzen, Direktappliziermittel, wie z.B. mikrokristalline Cellulose, Stärke und Stärkehydrolisate (z.B. Celutab®), Milchzucker, Polyethylenglykole, Polyvinylpyrrolidon und Dicalciumphosphat, Gleitmittel, Füllstoffe, wie z.B. Lactose oder Stärke, Bindemittel in Form von Lactose, Stärkearten, wie z.B. Weizen oder Mais bzw. Reisstärke, Cellulosederivate, wie z.B.

Methylcellulose, Hydroxypropylcellulose oder Kieselerde, Talkum, Stearate, wie z.B. Magnesiumstearat, Aluminiumstearat, Calciumstearat, Talk, siliconisierter Talk, Stearinsäure, Cetylalkohol, hydrierte Fette, können ebenfalls verwendet werden.

Zur herkömmlichen Applikation auf der Haut lassen sich die üblichen Emulsionen, Gele, Salben, Cremes oder mischphasige bzw. amphiphile Emulsionssysteme (Öl/Wasser-Wasser/Öl-Mischphase) sowie Liposomen und Transfersomen nennen.

Als Hilfs- bzw. Trägerstoffe eignen sich beispielsweise Natriumalginat als Gelbildner zur Herstellung einer geeigneten Grundlage oder Cellulosederivate, wie z.B. Guar- oder Xanthangummi, anorganische Gelbildner, wie z.B. Aluminiumhydroxide oder Bentonite (sogenannte thixotrope Gelbildner), Polyacrylsäurederivate, wie z.B. Carbopol®, Polyvinylpyrrolidon, mikrokristalline Cellulose oder Carboxymethylcellulose. Weiterhin kommen amphiphile nieder- und höhermolekulare Verbindungen, wie Phospholipide in Betracht. Die Gele können entweder als Hydrogele auf Wasserbasis oder als hydrophobe Organogele, beispielsweise auf Basis von Gemischen nieder- und hochmolekularer Paraffinkohlenwasserstoffe und Vaseline vorliegen.

Als Emulgatoren lassen sich anionische, kationische oder neutrale Tenside, beispielsweise Alkaliseifen, Metallseifen, Aminseifen, sulfurierte und sulfonierte Verbindungen, Invertseifen, hohe Fettalkohole, Partialfettsäurester des Sorbitans und Polyoxyethylensorbitans, Wollwachs, Lanolin oder andere synthetische Produkte zur Herstellung der Öl/Wasser - und/oder Wasser/Öl-Emulsionen einsetzen.

Die hydrophilen Organogele können beispielsweise auf Basis hochmolekularer Polyethylenglykole zubereitet werden. Diese gelartigen Formen sind abwaschbar. Als Lipide in Form fett- und/oder öl- und/oder wachsartiger Komponenten zur Herstellung der Salben, Cremes oder Emulsionen werden Vaseline, natürliche oder synthetische Wachse, Fettsäuren, Fettalkohole, Fettsäureester, zum Beispiel als Mono-, Di- oder Triglyceride, Paraffinöl oder vegetabilische Öle, gehärtetes Rizinusöl oder Kokosöl, Schweinefett, synthetische Fette, z.B. auf Capryl-, Capron-, Laurin- und Stearinsäurebasis, oder Triglyceridmischungen wie Miglycol® eingesetzt.

Zur Einstellung des pH-Wertes können osmotisch wirksame Säuren und Laugen, z.B. Salzsäure, Zitronensäure, Natronlauge, Kalilauge, Natriumhydrogencarbonat, ferner Puffersysteme, wie z.B. Citrat, Phosphat, Tris-Puffer oder Triethanolamin, verwendet werden.

Zur Erhöhung der Stabilität können noch Konservierungsmittel, z.B. Methyl- oder Propylbenzoat (Parabene) oder Sorbinsäure hinzugesetzt werden.

Als weitere topisch applizierbare Formen lassen sich Pasten, Puder oder Lösungen erwähnen. Die Pasten enthalten als konsistenzgebende Grundlage häufig lyophile und hydrophile Hilfsstoffe mit sehr hohem Feststoffanteil. Die Puder oder topisch applizierbaren Pulver können zur Erhöhung der Dispersität sowie des Fließ- und Gleitvermögens sowie zur Verhinderung von Agglomeraten, z.B. Stärkearten, wie Weizen- oder Reisstärke, flammendisperses Siliciumdioxid oder Kieselerden, die auch als Verdünnungsmittel dienen, enthalten.

Als transdermale Systeme lassen sich insbesondere Pflaster herstellen, die auf Basis verschiedener Schichten und/oder Mischungen geeigneter Hilfs- und Trägerstoffe den Wirkstoff in gesteuerter Weise über längere oder kürzere Zeiträume abzugeben vermögen. Bei der Herstellung derartiger transdermaler Systeme kommen zum Zwecke einer verbesserten und/oder beschleunigten Penetration die Membrandurchdringung erhöhende Substanzen bzw. Permeationspromotoren, wie z.B. Ölsäure, Arzone®, Adipinsäurederivate, Ethanol, Harnstoff, Propylglycol neben geeigneten Hilfs- und Trägerstoffen, wie Lösungsmitteln, polymeren Bestandteilen, z.B. auf Basis von Eutragit®, in Betracht.

Des weiteren können auch Injektabila verabreicht werden. Diese werden entweder in Form von Ampullen oder auch als sogenannte gebrauchsfertige Injektabila, z.B. als Fertigspritzen oder Einmalspritzen, daneben auch in Durchstechflaschen zur mehrmaligen Entnahme, hergerichtet. Die Verabreichung der Injektabila kann in Form der subkutanen (s.c.), intramuskulären (i.m.), intravenösen (i.v.) oder intrakutanen (i.c.) Applikation erfolgen. Die jeweils zweckmäßigen Injektionsformen können insbesondere als Lösungen, Kristallsuspensionen, nanopartikuläre oder kolloiddisperse Systeme, wie z.B. Hydrosole, hergestellt werden.

Die injizierbaren Zubereitungen können auch als Konzentrate hergestellt werden, welche mit wässrigen isotonischen Verdünnungsmitteln auf die gewünschte Wirkstoffdosierung eingestellt werden können. Weiterhin können sie auch als Pulver, wie z. B. Lyophylisate, hergestellt werden, die dann vorzugsweise unmittelbar vor der Applikation mit geeigneten Verdünnungsmitteln aufgelöst oder dispergiert werden.

Als Hilfs- und Trägerstoffe bei der Herstellung von injizierbaren Zubereitungen kommen *aqua sterilisata,* den pH-Wert beeinflussende Substanzen, wie z.B. organische und anorganische Säuren und Basen sowie deren Salze, Puffersubstanzen zur Einstellung des pH-Wertes, Isotonisierungsmittel, wie z.B. Natriumchlorid, Natriumhydrogencarbonat, Glucose und Fructose, Tenside bzw. oberflächenaktive Substanzen und Emulgatoren, wie z.B. Partialfettsäureester des Polyoxyethylensorbitans (Tween®), oder z.B. Fettsäureester des Polyoxyethylen (Cremophor®), fette Öle, wie z.B. Erdnussöl, Sojabohnenöl und Rizinusöl, synthetische Fettsäureester, wie z.B. Ethyloleat, Isopropylmyristat und Neutralöl (Miglycol®), sowie polymere Hilfsstoffe, wie z.B. Gelatine, Dextran, Polyvinylpyrrolidon, die Löslichkeit erhöhende Zusätze organischer Lösungsmittel, wie z.B. Propylenglycol, Ethanol, N,N-Dimethylacetamid, Propylenglycol oder komplexbildender Stoffe, wie z.B. Citraten und Harnstoff, Konservierungsmittel, wie z.B. Benzolsäurehydroxypropyl- und methylester, Benzylalkohol, Antioxidanten, wie z.B. Natriumsulfit und Stabilisatoren, wie z.B. EDTA, in Betracht.

Der Fachmann kann die jeweils geeigneten Arzneiformen in Einklang mit den Rezeptvorschriften und Verfahrensweisen auf Basis pharmazeutisch-physikalischer Grundlagen leicht erkennen und herstellen.

Als besonders vorteilhaft hat sich die Verwendung von Zileuton in der Form von Zyflo™Filmtab® Tabletten bewährt. Diese Substanz ist für die Prophylaxe von Asthma und Behandlung von chronischem Asthma von Erwachsenen und Kindern von 12 Jahren und älter indiziert und ist für diese Indikation in den USA zugelassen. Die Verwendung von Zileuton zur Herstellung des zyflo™Filmtab® Tabletten, wie sie von Abbott Laboraties in den USA vertrieben wird, zur Behandlung von Multiple Sklerose beim Menschen ist durch diese Erfindung mitumfaßt.

### Experimenteller Teil

In einem Behandlungsversuch wurden 15 Patienten mit klinisch aktiver MS mit Zileuton behandelt. Als Vergleich dienten 15 MS Patienten ohne diese Behandlung. Es wurde viermal täglich je eine Tablette Zyflo™Filmtab® verabreicht. Die Studie erstreckte sich über 3 Jahre. Im Vergleich zu 15 MS Patienten ohne diese Behandlung gingen Schubfrequenz und chronische Progredienz bei den mit Zileuton behandelten MS-Patienten signifikant zurück.

Zur Sicherung des Therapieeffekts wurden im Abstand von sechs Monaten Kernspinuntersuchungen des Gehirns unter Verwendung des Kontrastmittels Magnevist® durchgeführt, um die Floridität bzw. Akuität von Entzündungsherden zu objektivieren. Es konnten bei den mit Zileuton behandelten MS-Patienten keine neuen bzw. akuten Kontrastmittel-aufnehmenden Herde (Floride) festgestellt werden.

## Patentansprüche

1. Verwendung einer Verbindung der allgemeinen Formel I und/oder einem pharmazeutisch verträglichen Salz davon als aktiven Wirkstoff für die Herstellung einer pharmazeutischen Zusammensetzung für die Behandlung von MS beim Menschen: 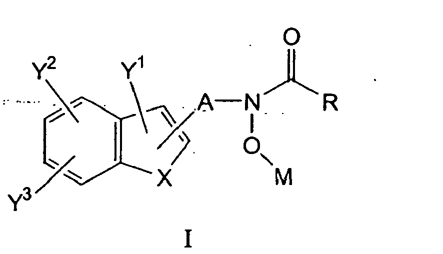 worin:
X: NCH₃, O oder S bedeutet,
A: unsubstituiertes C₁₋₄-Alkylen oder substituiertes C₁₋₄-Alkylen, das einfach oder mehrfach mit Substituenten unabhängig ausgewählt aus CH₃, CH₂CH₃, CH₂CH₂CH₃ und CH(CH₃)₂ substituiert ist, bedeutet,
M: H; Benzoyl; unsubstituiertes C₁₋₁₂-Alkanoyl; substituiertes C₁₋₁₂-Alkanoyl, das einfach oder mehrfach mit Substituenten unabhängig ausgewählt aus CH₃, CH₂CH₃, F und Cl, substituiert ist; unsubstituiertes C₁₋₁₂-Alkyl; oder substituiertes C₁₋₁₂-Alkyl, das einfach oder mehrfach mit Substituenten unabhängig ausgewählt aus CH₃, CH₂CH₃, F und Cl, substituiert ist; bedeutet,
R: CH₃, CH₂CH₃ oder NR¹R² bedeutet, worin R¹ und R² unabhängig voneinander H, CH₃ oder CH₂CH₃ bedeuten, und
Y¹, Y² und Y³: unabhängig voneinander ausgewählt sind aus H, CH₃, CH₂CH₃, F und Cl.

2. Verwendung gemäß Anspruch 1, worin:
M: H bedeutet.

3. Verwendung gemäß Anspruch 1 oder 2, worin:
mindestens zwei der Y¹, Y² und Y³: H bedeuten.

4. Verwendung gemäß einem der Ansprüche 1 bis 3, worin:
A: CH₂ oder CHCH₃ bedeutet.

5. Verwendung gemäß einem der Ansprüche 1 bis 4, worin:
X: S bedeutet.

6. Verwendung gemäß einem der Ansprüche 1 bis 5, worin die Verbindung der allgemeinen Formel I ausgewählt ist aus:
N-Hydroxy-N- (1-benzo [b] thien-2-ylethyl) -acetamid,
1-(1-Benzo[b]thien-2-ylethyl)-1-hydroxyharnstoff,
N-Hydroxy-N-(1-benzo[b]thien-2-ylethyl)-N'-methyl-harnstoff, N-Hydroxy-N-(1-benzo[b]thien-2-ylethyl)-N', N'-dimethyl-harnstoff,
N-Hydroxy-N-benzo[b]thien-2-ylmethyl-harnstoff,
N-Hydroxy-N-benzo[b]thien-2-ylmethyl-N'-methyl-harnstoff,
N-Hydroxy-N-benzo[b]thien-2-ylmethyl-N',N'-dimethyl-harnstoff,
N-Hydroxy-N-(1-benzo[b]thien-3-ylethyl)-acetamid,
N-Hydroxy-N-(1-benzo[b]thien-3-ylethyl)-harnstoff,
N-Hydroxy-N-[1-(3-methylbenzo[b]thien-2-yl)-ethyl]-harnstoff, und
N-Hydroxy-N-[1-(5-fluorbenzo[b]thien-2-yl)-ethyl]-harnstoff.

7. Verwendung gemäß einem der vorangehenden Ansprüche, worin die pharmazeutische Zusammensetzung außerdem einen oder mehrere pharmazeutisch verträgliche Träger und/oder einen oder mehrere toxikologisch unbedenkliche Hilfsstoffe umfaßt.

8. Verwendung gemäß einem der vorangehenden Ansprüche, worin das Arzneimittel weitere Wirkstoffe umfaßt.

9. Verwendung gemäß einem der vorangehenden Ansprüche, worin das Arzneimittel in einer der folgenden Formen hergestellt wird: in Form eines Inhalationstherapeutikums, in Form eines transdermalen therapeutischen Systems, in Form eines gastrointestinalen therapeutischen Systems, als Tablette, Kapsel, Dragee, Lösung, Suspension, Brausetablette, Salbe, Suspension, Emulsion, Balsam, Pflaster oder Dosieraerosol.

10. Verwendung gemäß einem der vorangehenden Ansprüche, worin die Konzentration des aktiven Wirkstoffs pro Dosis 0,001 bis 2,0 mg beträgt.

11. Verwendung gemäß Anspruch 10, worin die Konzentration des aktiven Wirkstoffs pro Dosis 0,01 bis 2,0 mg beträgt.

12. Verwendung gemäß Anspruch 10, worin die Konzentration des aktiven Wirkstoffs pro Dosis 0,1 bis 1,0 mg beträgt.

13. Verwendung gemäß einem der vorangehenden Ansprüche, worin das Arzneimittel zur ein- bis fünfmal täglichen Gabe hergestellt wird.

## Claims

1. Use of a compound of the general formula I and/or a pharmaceutically acceptable salt thereof as active ingredient for the preparation of a pharmaceutical composition for the treatment of multiple sclerosis in humans: 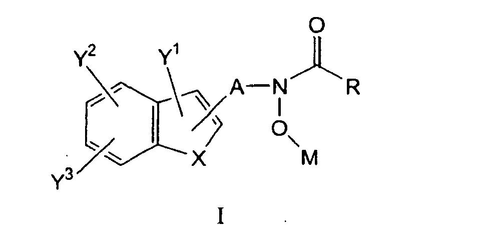 wherein
X represents NCH₃, O or S,
A represents an unsubstituted C₁₋₄ alkylene or a substituted C₁₋₄ alkylene that is mono- or polysubstituted with substituents independently selected from CH₃, CH₂CH₃, CH₂CH₂CH₃ und CH(CH₃)₂,
M represents H; benzoyl; unsubstituted C₁₋₁₂ alkanoyl; substituted C₁₋₁₂ alkanoyl that is mono- or polysubstituted with substituents independently selected from CH₃, CH₂CH₃, F and Cl; unsubstituted C₁₋₁₂ alkyl; or substituted C₁₋₁₂ alkyl that is mono- or polysubstituted with substituents independently selected from CH₃, CH₂CH₃, F and Cl;
R represents CH₃, CH₂CH₃ or NR¹R², wherein R¹ and R² independently represent H, CH₃ or CH₂CH₃, and
Y¹, Y² and Y³ are independently selected from H, CH₃CH₂CH₃, F and Cl.

2. Use according to claim 1, wherein M represents H.

3. Use according to claim 1 or 2, wherein at least two of Y¹, Y² and Y³ represent H.

4. Use according to any one of claims 1 to 3, wherein A represents CH₂ or CHCH₃.

5. Use according to any one of claims 1 to 4, wherein X represents S.

6. Use according to any one of claims 1 to 5, wherein the compound of the general formula I is selected from:
N-hydroxy-N-(1-benzo[b]thien-2-ylethyl) acetamide,
1-(1-benzo[b]thien-2-ylethyl)-1-hydroxy urea,
N-hydroxy-N-(1-benzo[b]thien-2-ylethyl)-N'-methyl urea,
N-hydroxy-N-(1-benzo[b]thien-2-ylethyl)-N',N'-dimethyl urea,
N-hydroxy-N-benzo[b]thien-2-ylmethyl urea,
N-hydroxy-N-benzo[b]thien-2-ylmethyl-N'-methyl urea,
N-hydroxy-N-benzo[b]thien-2-ylmethyl-N',N'-dimethyl urea,
N-hydroxy-N=(1-benzo[b]thien-3-ylethyl) acetamide,
N-hydroxy-N-(1-benzo[b]thien-3-ylethyl) urea,
N-hydroxy-N- [1- (3-methylbenzo [b] thien-2-yl) -ethyl] urea, and
N-hydroxy-N- [1- (5-fluorobenzo [b] thien-2-yl) -ethyl] urea.

7. Use according to any one of the preceding claims, wherein the pharmaceutical composition additionally comprises one or more pharmaceutically acceptable carriers and/or one or more toxicologically safe adjuvants.

8. Use according to any one of the preceding claims, wherein the medicament comprises further active ingredients.

9. Use according to any one of the preceding claims, wherein the medicament is prepared in one of the following forms: in the form of an inhalation therapeutic agent, in the form of a transdermal therapeutic system, in the form of a gastro-intestinal therapeutic system, as tablet, capsule, dragée, solution, suspension, effervescent tablet, ointment, suspension, emulsion, balm, plaster or dosage aerosol.

10. Use according to any one of the preceding claims, wherein the concentration of the active ingredient amounts to 0.001 to 2.0 mg per dose.

11. Use according to claim 10, wherein the concentration of the active ingredient amounts to 0.1 to 2.0 mg per dose.

12. Use according to claim 10, wherein the concentration of the active ingredient amounts to 0.1 to 1.0 mg per dose.

13. Use according to any one of the preceding claims, wherein the medicament is prepared for one to five daily dosages.

## Revendications

1. Utilisation d'un composé de formule générale I et/ou d'un sel pharmaceutiquement compatible de celui-ci, en tant que principe actif pour la préparation d'une composition pharmaceutique pour le traitement des MS (scléroses multiples) chez l'homme ; 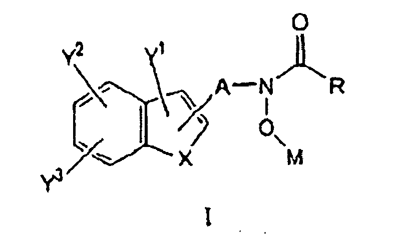 dans laquelle :
X signifie : NCH₃, O ou S,
A signifie : alkylène en C_{1à4} non substitué ou alkylène enC_{1à4} substitué, substitué individuellement ou en plusieurs exemplaires, indépendamment, avec des substituants parmi CH₃, CH₂CH₃, CH₂CH₂CH₃ et CH(CH₃)₂,
M signifie : H ; benzoyle; alkanoyle en C_{1à12} non substitué ; alkanoyle en C_{1à12} substitué, substitué, individuellement ou en plusieurs exemplaires, indépendamment, avec des substituants parmi CH₃, CH₂CH₃, F et C1, ; alkyl en C_{1à12} non substitué ou alkyl en C_{1à12} substitué, substitué individuellement ou en plusieurs exemplaires indépendamment avec des substituants parmi CH₃, CH₂CH₃, F et Cl,
R signifie : CH₃, CH₂CH₃ ou NR¹R², où R¹ et R² signifient, indépendamment l'un de l'autre, H, CH₃ ou CH₂CH₃, et
Y¹, Y² et Y³: sont choisis indépendamment les uns les autres, parmi H, CH₃, CH₂CH₃, F et Cl.

2. Utilisation selon la revendication 1, dans laquelle :
M signifie : H.

3. Utilisation selon la revendication 1 ou 2, dans laquelle :
au moins deux parmi Y¹, Y² et Y³ signifient : H.

4. Utilisation selon l'une des revendications 1 à 3, dans laquelle :
A signifie : CH₂ ou CHCH₃.

5. Utilisation selon l'une des revendications 1 à 4, dans laquelle :
X signifie : S.

6. Utilisation selon l'une des revendications 1 à 5, dans laquelle la combinaison de formule générale I est sélectionnée parmi :
N-hydroxy-N-(1-benzo [b]thiène-2-yléthyl)-acétamide,
1-(1-benzo[b]thiène-2-yléthyl)-1-hydroxyurée,
N-hydroxy-N-(1-benzo[b]thiène-2-yléthyl)-N'-méthyl-urée,
N-Hydroxy-N-(1-benzo[b]thiène-2-yléthyl)-N', N'-diméthyl-urée,
N-hydroxy-N-benzo[b]thiène-2-ylméthyl-urée,
N-hydroxy-N-benzo[b]thiène-2-ylméthyl-N'-méthyl-urée,
N-hydroxy-N-benzo [b]thiène-2-ylméthyl-N', N' -diméthylurée,
N-hydroxy-N-(1-benzo [b]thiène-3-yléthyl)-acétamide,
N-hydroxy-N-(1-benzo[b]thiène-3-yléthyl)-urée,
N-Hydroxy-N-[1-(3-méthylbenzo[b]thiène-2-yl)-éthyl]-urée, et
N-Hydroxy-N-[1-(5-fluorbenzo[b]thiène-2-yl)-éthyl]-urée.

7. Utilisation selon l'une des revendications précédentes, dans laquelle la composition pharmaceutique comprend en outre un ou plusieurs porteurs pharmaceutiquement compatibles et/ou une ou plusieurs substances auxiliaires inoffensives toxicologiquement.

8. Utilisation selon l'une des revendications précédentes, dans laquelle le médicament pharmaceutique comprend d'autres principes actifs.

9. Utilisation selon l'une des revendications précédentes, dans laquelle le médicament est préparé selon l'une des formes suivantes : sous forme d'un produit thérapeutique par inhalation, sous forme d'un système thérapeutique transdermique, sous forme d'un système thérapeutique gastro-intestinal, sous forme de comprimés, de capsules, de dragées, de solutions, de suspensions, de comprimés effervescents, de pommade, une suspension, une émulsion, un baume, un plâtre ou un aérosol à dosage.

10. Utilisation selon l'une des revendications précédentes, dans laquelle la concentration du principe actif par dose est de 0,001 à 2, 0 mg.

11. Utilisation selon la revendication 10, la concentration du principe actif par dose étant de 0,01 à 2,0 mg.

12. Utilisation selon la revendication 10, dans laquelle la concentration du principe actif par dose est de 0,1 à 1,0 mg.

13. Utilisation selon l'une des revendications précédentes, dans laquelle le médicament est préparé pour une administration de une à cinq fois par jour.
